(19) Europäisches Patentamt
European Patent Office
Office européen des brevets

(11) EP 0 366 061 B1

(12) EUROPÄISCHE PATENTSCHRIFT

(45) Veröffentlichungstag und Bekanntmachung des Hinweises auf die Patenterteilung:
**17.01.1996 Patentblatt 1996/03**

(51) Int Cl.[6]: **C07D 405/04**, C07D 405/14, C07D 409/14, A61K 31/35, A61K 31/445

(21) Anmeldenummer: **89119710.5**

(22) Anmeldetag: **24.10.1989**

# (54) Die Verwendung von 4H-1-Benzopyran-4-on-Derivaten, neue 4H-1-Benzopyran-4-on-Derivate und diese enthaltende Arzneimittel

Use of 4H-1-benzopyran-4-one derivatives, 4H-1-benzopyran-4-one derivatives and medicines containing same

Application de dérivés de la 4H-1-benzopyrannone-4, dérivés de la 4H-1-benzopyrannone-4 et médicaments les contenant

(84) Benannte Vertragsstaaten:
**AT BE CH DE ES FR GB GR IT LI LU NL SE**

(30) Priorität: **28.10.1988 DE 3836676**

(43) Veröffentlichungstag der Anmeldung:
**02.05.1990 Patentblatt 1990/18**

(73) Patentinhaber:
**HOECHST AKTIENGESELLSCHAFT**
**D-65926 Frankfurt am Main (DE)**

(72) Erfinder:
- **Naik, Ramchandra Ganapati, Dr.**
  **Mulund (West), Bombay 400 082 (IN)**
- **Lal, Bansi, Dr.**
  **Mulund (West), Bombay 400 080 (IN)**
- **Rupp, Richard Helmut, Dr.**
  **D-6240 Königstein/Taunus (DE)**
- **Sedlacek, Hans Harald, Dr.**
  **D-3550 Marburg (DE)**
- **Dickneite, Gerhard, Dr.**
  **D-3550 Marburg (DE)**
- **Czech, Jörg, Dr.**
  **D-3550 Marburg (DE)**

(56) Entgegenhaltungen:
**DE-A- 3 329 186** **DE-A- 3 612 337**

- **CHEMICAL ABSTRACTS, Band 108, Nr. 21, 23. Mai 1988, Columbus, Ohio, USA, ALAM,MAKTOOB et al.: "Tubastraine: isolation and structure of a novel alkaloid from the stony coral Tubastraea micrantha", Seite 438, Spalte 1, Zusammenfassung-Nr. 183888m.**
- **CHEMICAL ABSTRACTS, Band 106, Nr. 6, 9. Februar 1987, Columbus, Ohio, USA; BHAT,SUJATA VASUDEV et al.: "Isolation of a pharmacologically active substancehaving the structure of a chromone alkaloid from plant(s) belonging to theMeliaceae family", Seite 359, Spalte 1, Zu-sammenfassung-Nr. 38467c**

Bemerkungen:
Die Akte enthält technische Angaben, die nach dem Eingang der Anmeldung eingereicht wurden und die nicht in dieser Patentschrift enthalten sind.

EP 0 366 061 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die vorliegende Erfindung betrifft die Verwendung von 4H-1-Benzopyran-4-on-Derivaten, neue 4H-1-Benzoypran-4-on-Derivate und diese enthaltende Arzneimittel.

Benzopyranderivate sind bereits aus der europäischen Patentschrift mit der Nr. 0137193 und der deutschen Offenlegungsschrift 36 12 337 bekannt.In letzterer werden Verbindungen der Formel a) offenbart

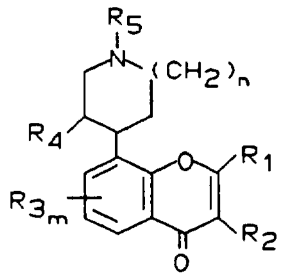

a)

in der die Substituenten $R_1$ bis $R_5$ und m sowie n die genannten Bedeutungen haben; die Verbindungen haben eine entzündungshemmende analgetische und immunmodulierende Wirkung.

Überraschenderweise wurde nun gefunden, daß bestimmte 4H-1-Benzopyran-4-on-Derivate Onkogen-kodierte Kinasen inhibieren und sich somit zur Bekämpfung von Tumorerkrankungen eignen.

Die Expression von Onkogenen in einer Säuretierzelle geht mit dem Übergang vom normalen zum transformierten Zelltypus, welcher dann zu einer Krebszelle wird, einher. Die Transformation wurde durch Infektion einer Zelle mit einem Retrovirus hervorgerufen. Ein gut bekanntes Beispiel war die Infektion von Hühnern mit Rous-Sarkom-Virus, welche anschließend Krebs entwickelten. Das entsprechende Onkogen, das für die bösartige Transformation verantwortlich war, wurde mit "SRC"-(Sarcoma) Gen bezeichnet (J.S. Brugge, R.L. Erikson; Nature 269, 346-348 (1977)). Viele bis dato bekannte Onkogene sind durch die Expression eines Proteins mit Kinaseaktivität gekennzeichnet. Die Enzyme katalysieren die Übertragung der endständigen Phosphatgruppe des ATP auf eine Aminosäure. Im Gegensatz zu vielen anderen Proteinkinasen, die die Phosphatgruppe auf einen Seryl- oder Threonylrest übertragen, phosphorylieren die meisten Onkogen-kodierten Kinasen einen Tyrosylrest der Proteinkette. Abgesehen davon ist es bekannt, daß Produkte von Onkogenen, nämlich die des v-mos-, v-mil- und v-raf- Onkogens, Serin/Threonin-spezifische Proteinkinaseaktivität besitzen. (K. Mölling et al., Nature (London) 312, 558-561 (1984); B. Singh et al., Journal of Virology 60, 1149-1152 (1986)).

Tyrosinkinaseaktivität wird auch in Wachstumsfaktorrezeptoren exprimiert; neue Erkenntnisse zeigen nun, daß das Wachstum vieler Tumoren von der Gegenwart von Wachstumsfaktoren abhängt, wie Epidermal Growth Factor (EGF), Transforming Growth Factor α (TGFα) oder Platelet Derived Growth Factor (POGF) (A.S. Goustin, G.D. Shipley, H.L. Moses, Cancer Research 46, 1015-1029 (1986)). Im Anschluß an die Bindung des Wachstumsfaktors an seinen Rezeptor wird die Tyrosinkinase, welche eine Eigenkomponente des Wachstumsfaktorrezeptors ist, stimuliert.

Von einem Inhibitor der Tyrosinkinase und vielleicht der Serin/Threoninkinase könnte deshalb die Inhibierung von Tumorwachstum und Tumorausbreitung erwartet werden, und er könnte in der Tumortherapie eingesetzt werden.

Die vorliegende Erfindung betrifft deshalb die Verwendung von 4H-1-Benzopyran-4-on-Derivaten der Formel I zur Herstellung eines Arzneimittels mit einer Wirkung gegen Tümoren. Die Formel I lautet,

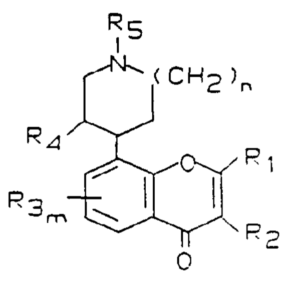

I

in der

$R_1$ Wasserstoff, Alkyl mit 1 bis 6 Kohlenstoffatomen, Aryl-$C_1$-$C_4$-alkyl, substituiertes $C_1$-$C_6$-Alkyl, $C_3$-$C_6$-Cycloalkyl, einen $C_3$-$C_9$-Heterocyclus mit 1, 2 oder 3 Heteroatomen wie N, S, O oder deren beliebige Kombinationen, $C_3$-$C_6$-Cycloalkyl-$C_1$-$C_4$-alkyl, $C_2$-$C_6$-Alkenyl, $C_2$-$C_6$-Alkinyl, Aryl, polycyclische Ringe einbegriffen, aromatische heterocyclische Reste, substituiertes Aryl, Carboxyl oder eine Aldehyd- oder COO-$C_1$-$C_4$-Alkylgruppe, eine primäre Amino-, Alkylamino-, Aralkylamino-, Dialkylamino-, Amido-, Arylamino-, Diarylaminogruppe oder

$-CH_2O-C_1-C_4$-Alkyl bedeutet;
$R_2$ Wasserstoff, oder $C_1-C_3$ Alkyl bedeutet;
$R_3$ gleich ist und OH oder $OCH_3$ bedeutet,
$R_4$ Hydroxy, bedeutet;
$R_5$ $CH_3$ bedeutet;
m gleich der Zahl 2 ist und
n gleich der Zahl 1 ist,

und deren pharmakologisch unbedenkliche Säureadditionssalze.

Die erfindungsgemäßen Verbindungen haben zwei asymmetrische Zentren, eines an der Verbindungsstelle des Stickstoffheterocyclischen Ringes mit dem Benzopyranteil (C-4'), das andere beim durch $R_4$ substituierten Kohlenstoffatom (C-3'), wodurch zwei optische Isomerenpaare möglich sind. Die Definition der erfindungsgemäßen Verbindungen beeinhaltet alle möglichen Stereoisomere und deren Mischungen. Ganz besonders beeinhaltet sie die racemischen Formen und die isolierten optischen Isomeren mit der angegebenen Aktivität. Die zwei Racemate können durch physikalische Methoden, wie z.B. fraktionierte Kristallisation, getrennt werden. Die einzelnen optischen Isomere können von den Racematen durch Standardmethoden, wie z.B. Salzbildung mit einer optisch aktiven Säure und anschließende Kristallisation, erhalten werden.

Geeignete Alkylgruppen für $R_1$ sind z.B. geradkettige oder verzweigte Radikale mit bis zu 6, vorzugsweise bis zu 5 Kohlenstoffatomen, z.B. Methyl-, Äthyl-, Propyl-, Isopropyl-, t-Butyl-, Pentyl- oder Isopentylgruppen.

Geeignete substituierte Alkylgruppen für $R_1$ sind z.B. Halogenalkyl, wie Trifluormethyl, Hydroxyalkyl, wie Hydroxyäthyl, oder Carboxyalkyl, wie Carboxyäthyl.

Geeignete Beispiele für eine Cycloalkylgruppe als $R_1$ mit 3 bis 6 Kohlenstoffatomen sind Cyclopropyl, Cyclobutyl, Cyclopentyl oder Cyclohexyl. Cyclopropylmethyl ist ein Beispiel für Cycloalkylalkyl.

Ein Beispiel für eine Aralkylgruppe als $R_1$ ist eine Phenylalkylgruppe, in der die Phenylgruppe unsubstituiert oder einfach oder mehrfach durch Substituenten wie Halogen, $C_1-C_4$-Alkyl, $C_1-C_4$-Alkoxy, Nitro oder eine Trifluormethylgruppe, Aminogruppe und substituierte Aminogruppe substituiert ist.

Ein Beispiel für eine Arylgruppe als $R_1$ ist eine Phenylgruppe, die unsubstituiert oder einfach oder mehrfach durch Substituenten wie Halogen, $C_1-C_4$-Alkyl, $C_1-C_4$-Alkoxy, Hydroxy, Carboxy, Coo-Alkyl, $CONH_2$, CONH-Alkyl, CON $(Alkyl)_2$, Nitro oder Trifluormethyl, Amino, $C_1-C_4$-Alkylamino, Di-$C_1-C_4$-alkylamino, aromatische Heterocyclen wie Pyridylgruppen und polycyclische aromatische Reste wie Naphthylgruppen substituiert ist.

Ein geeignetes Beispiel für eine Alkylaminogruppe als $R_1$ ist $(CH_2)_n-NR_6R_7$, wobei n 1 bis 3 ist und $R_6$ und $R_7$ Alkyl sind und dieselbe Bedeutung haben wie oben für Alkyl $R_1$ bis $R_5$ angegeben; außerdem können $R_6$ und $R_7$ gemeinsam mit dem Stickstoffatom, an das sie gebunden sind, ein heterocyclischer Ring mit einem oder mehreren Heteroatomen sein. Geeignete Beispiele für heterocyclische Ringe, die von $R_6$ und $R_7$, gemeinsam mit dem Stickstoff, an den sie gebunden sind, gebildet werden, sind Piperidin, Pyrrolidin, Morpholin, Piperazin oder Imidazol, die unsubstituiert oder in einer oder mehreren Stellungen durch $C_1-C_4$-Alkyl, $C_1-C_4$-Alkoxy, Aryl oder eine Hydroxyl- oder Aminogruppe substituiert sein können.

Geeignete Beispiele für Salze der erfindungsgemäßen Verbindungen mit anorganischen oder organischen Säuren sind das Hydrochlorid, Hydrobromid, Sulfat, Phosphat, Acetat, Oxalat, Tartrat, Citrat, Maleat oder Fumarat. Bevorzugt ist die Verwendung von Verbindungen der Formel Ia

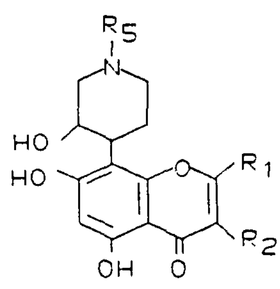

Ia

in der $R_1$ Wasserstoff oder $C_1-C_3$-Alkyl, Naphthyl, Aryl, Aralkyl, substituiertes Aryl oder einen $C_3-C_9$-Heterocyclus bedeutet, oder deren pharmakologisch unbedenkliche Säureadditionssalzen.

Ganz besonders bevorzugt ist die Verwendung von Verbindungen der Formel Ia gemäß Anspruch 2, die dadurch gekennzeichnet sind, daß $R^1$ Phenyl, Thienyl, Pyridyl, Chlorphenyl, Dichlorphenyl, Methylphenyl, Aminophenyl, Bromphenyl, Hydroxyphenyl oder Naphthyl bedeutet,
$R_2$ Wasserstoff bedeutet, und

$R_5$ Methyl bedeutet oder deren pharmakologisch unbedenklichen Säureadditionssalzen.

Die Herstellung dieser Verbindungen kann z.B. wie in der EP 0 157 193 oder der DE 3 612 337 beschrieben erfolgen.

Bevorzugte. Verbindungen, die sich für die erfindungsgemäße Verwendung besonders eignen sind z.B.:

(±)-cis-5,7-Dihydroxy-2-(2-thienyl)-8-[4-(3-hydroxy-1-methyl)piperidinyl]-4H-1-benzopyran-4-on,
(-)-cis-5,7-Dihydroxy-2-phenyl-8-[4-(3-hydroxy-1-methyl)-piperidinyl]-4H-1-benzopyran-4-on-hydrochlorid,
(±)-cis-5,7-Dihydroxy-2-phenyl-8-[4-(3-hydroxy-1-methyl)-piperidinyl]-4H-1-benzopyran-4-on-hydrochlorid,
(+)-cis-5,7-Dihydroxy-2-phenyl-8-[4-(3-hydroxy-1-methyl)-piperidinyl]-4H-1-benzopyran-4-on-hydrochlorid,
(-)-cis-5,7-Dihydroxy-2-(2-pyridyl)-8-[4-(3-hydroxy-1-methyl)piperidinyl]-4H-1-benzopyran-4-on-hydrochlorid,
(±)-cis-5,7-Dihydroxy-2-(2-pyridyl)-8-[4-(3-hydroxy-1-methyl)piperidinyl]-4H-1-benzopyran-4-on-hydrochlorid,
(±)-cis-5,7-Dihydroxy-2-(3-pyridyl)-8-[4-(3-hydroxy-1-methyl)piperidinyl]-4H-1-benzopyran-4-on-hydrochlorid,
(±)-cis-5,7-Dihydroxy-2-(4-pyridyl)-8-[4-(3-hydroxy-1-methyl)piperidinyl]-4H-1-benzopyran-4-on-hydrochlorid,
(-)-cis-5,7-Dihydroxy-2-(2-chlorphenyl)-8-[4-(3-hydroxy-1-methyl)piperidinyl]-4H-1-benzopyran-4-on-hydrochlorid,
(+)-cis-5,7-Dihydroxy-2-(2-chlorphenyl)-8-[4-(3-hydroxy-1-methyl)piperidinyl]-4H-1-benzopyran-4-on-hydrochlorid,
(±)-cis-5,7-Dihydroxy-2-(2-chlorphenyl)-8-[4-(3-hydroxy-1-methyl)piperidinyl]-4H-1-benzopyran-4-on-hydrochlorid,
(±)-cis-5,7-Dihydroxy-2-(2,5-dichlorphenyl)-8-[4-(3-hydroxy-1-methyl)piperidinyl]-4H-1-benzopyran-4-on-hydrochlorid,
(±)-cis-5,7-Dihydroxy-2-(2,4-dichlorphenyl)-8-[4-(3-hydroxy-1-methyl)piperidinyl]-4H-1-benzopyran-4-on-hydrochlorid,
(±)-cis-5,7-Dihydroxy-2-(4-methylphenyl)-8-[4-(3-hydroxy-1-methyl)piperidinyl]-4H-1-benzopyran-4-on-hydrochlorid,
(±)-cis-5,7-Dihydroxy-2-(3-methylphenyl)-8-[4-(3-hydroxy-1-methyl)piperidinyl]-4H-1-benzopyran-4-on-hydrochlorid,
(±)-cis-5,7-Dihydroxy-2-(4-aminophenyl)-8-[4-(3-hydroxy-1-methyl)piperidinyl]-4H-1-benzopyran-4-on-hydrochlorid,
(±)-cis-5,7-Dihydroxy-2-(3-bromphenyl)-8-[4-(3-hydroxy-1-methyl)piperidinyl]-4H-1-benzopyran-4-on-hydrochlorid,
(±)-cis-5,7-Dihydroxy-2-(2-naphthyl)-8-[4-(3-hydroxy-1-methyl)piperidinyl]-4H-1-benzopyran-4-on-hydrochlorid,
(±)-cis-5,7-Dihydroxy-2-(4-hydroxyphenyl)-8-[4-(3-hydroxy-1-methyl)piperidinyl]-4H-1-benzopyran-4-on und
(±)-cis-5,7-Dihydroxy-2-(2-hydroxyphenyl)-8-[4-(3-hydroxy-1-methyl)piperidinyl]-4H-1-benzopyran-4-on.

Weitere erfindungsgemäße 4H-1-Benzopyran-4-on-Derivate bzw. Verbindungen, die sich besonders für die erfindungsgemäße Verwendung eignen, sind in den nachfolgenden Tabellen 1 und 2 mit zugehörigen physikalischen Daten aufgeführt. Die Tabelle 1 bezieht sich auf die Formel Ic

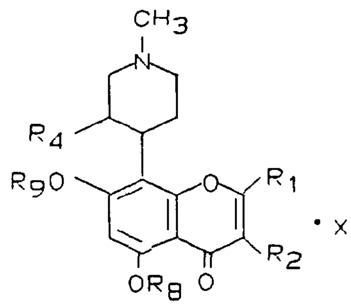

Ic

und die Tabelle 2 bezieht sich auf die Formel Id

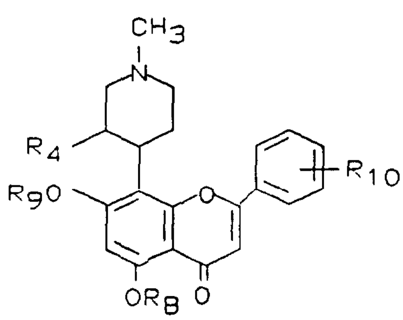

Id

Tabelle 1

| Verb. | $R_1$ | $R_2$ | $R_4$ | $R_8$ | $R_9$ | X | Schmelzpunkt (°C) | Optische Drehung |
|---|---|---|---|---|---|---|---|---|
| 1 | H | H | OH | H | H | - | 298 (Zers.) | (±) |
| 2 | H | H | OH | $CH_3$ | $CH_3$ | HCl, 1,5$H_2O$ | 173-175 | (±) |
| 3 | $CH_3$ | H | OH | H | H | HCl, | 237-240 | (±) |
| 4 | $CH_3$ | H | OH | H | H | HCl, | 241-43 | (+) |
| 5 | $CH_3$ | H | OH | H | H | HCl, | 241-42 | (-) |
| 6 | $CH_3$ | H | OH | H | $CH_3$ | HCl, | 230-232 | (±) |
| 7 | $CH_3$ | H | OH | $CH_3$ | $CH_3$ | 2HCl, 2$H_2O$ | 236-239 | (±) |
| 9 | $C_2H_5$ | H | OH | H | H | HCl, 1,5$H_2O$ | 230-233 | (±) |
| 10 | $C_2H_5$ | H | OH | $CH_3$ | $CH_3$ | HCl, 1,5$H_2O$ | 240-242 | (±) |
| 11 | n-$C_3H_7$ | $CH_3$ | OH | H | H | - | 191-192 | (±) |
| 12 | n-$C_3H_7$ | H | OH | H | H | HCl, | 190-192 | (±) |
| 13 | n-$C_3H_7$ | H | OH | H | H | HCl, 0,5$H_2O$ | 197-200 | (±) |
| 14 | n-$C_3H_7$ | H | OH | H | H | HCl, 0,5$H_2O$ | 198-201 | (-) |
| 15 | n-$C_4H_9$ | H | OH | H | H | HCl, $H_2O$ | 157-159 | (±) |
| 16 | $CH_3$ | $CH_3$ | OH | H | H | $H_2O$ | 232-233 | (±) |
| 17 | 2-Pyridyl | H | OH | H | H | HCl, 0,5$H_2O$ | 229°C | (±) |
| 18 | 3-Pyridyl | H | OH | H | H | 2HCl, 2$H_2O$ | 278-280 | (±) |
| 19 | 4-Pyridyl | H | OH | H | H | 2HCl, 1,5$H_2O$ | 236-238 | (±) |
| 20 | $CO_2H$ | H | OH | $CH_3$ | $CH_3$ | $H_2O$ | >340 | (±) |
| 21 | 2-Thienyl | H | OH | H | H | 2$H_2O$ | 243-244 | (±) |
| 22 | 2-Thienyl | H | OH | $CH_3$ | $CH_3$ | - | 207-208 | (±) |
| 23 | 2-Pyridyl | H | OH | H | H | 2HCl, 2$H_2O$ | 220-228 | (-) |
| 24 | β-Styryl | H | OH | H | H | HCl, 1,5$H_2O$ | >300 | (±) |
| 25 | 1-Naphthyl | H | OH | H | H | HCl, $H_2O$ | 195-200 | (±) |
| 26 | 2-Naphtyl | H | OH | H | H | HCl, 0,5$H_2O$ | 280-282 | (±) |
| 27 | (2-Chlorphenyl)methyl | H | OH | H | H | HCl | 270-275 | (±) |

Tablle 2

| Verb. | $R_{10}$ | $R_8$ | $R_9$ | X | Schmelzpunkt (°C) | Optische Drehung |
|---|---|---|---|---|---|---|
| 28 | H | H | H | HCl, $2H_2O$ | 273-275 | (±)/trans |
| 29 | 4-$NO_2$ | H | H | HCl, $3H_2O$ | 249 (Zers.) | (±) |
| 30 | 4-$NO_2$ | $CH_3$ | $CH_3$ | HCl, $2H_2O$ | 257-260(Zers.) | (±) |
| 31 | 2-Cl | H | H | HCl, $H_2O$ | 198-200 | (±) |
| 32 | 2-Cl | $CH_3$ | $CH_3$ | 1,5HCl, $H_2O$ | 190-191 | (±) |
| 33 | 4-$NH_2$ | H | H | 2HCl, $2H_2O$ | 240-242 | (±) |
| 31 | 3,5-Dimethoxy | $CH_3$ | $CH_3$ | 2HCl, $2H_2O$ | 180-182 | (±) |
| 32 | 4-Br | H | H | HCl, $2H_2O$ | 215 | (±) |
| 33 | 4-Cl | H | H | HCl, $1{,}5H_2O$ | 225 | (±) |
| 34 | 2,4-Dichlor | H | H | HCl, $2{,}5H_2O$ | 165-166 | (±) |
| 35 | 4-F | H | H | HCl, $H_2O$ | 285-287 | (±) |
| 36 | 2-F | H | H | HCl, $2H_2O$ | 263-265 | (±) |
| 37 | 4-Methyl | H | H | HCl, $1{,}5H_2O$ | 247-49 | (±) |
| 38 | 3,5-Dihydroxy | H | H | HCl, $3H_2O$ | 300-302 | (±) |
| 39 | 3-Cl | H | H | HCl, $2H_2O$ | 288-290 | (±) |
| 40 | 3-Methyl | H | H | HCl, $2H_2O$ | 268 | (±) |
| 41 | 2-Methyl | H | H |  | 204-205 | (±) |
| 42 | 2-Cl | H | H | HCl, $2H_2O$ | 190-192 | (+) |
| 43 | H | H | H | HCl | 269-271 | (±) |
| 44 | 3-Br | H | H | HCl, $2H_2O$ | 285 | (±) |
| 45 | 3-$CO_2$Me | $CH_3$ | $CH_3$ | 1,5HCl, $3H_2O$ | 235 | (±) |
| 46 | 2,5-Dichlor | H | H | HCl, $H_2O$ | 251-252 | (±) |
| 47 | 3-COOH | $CH_3$ | $CH_3$ | HCl, $1{,}5H_2O$ | 270 | (±) |
| 48 | 2-Cl | H | H | HCl, $1{,}5H_2O$ | 190-194 | (-) |
| 49 | H | H | H | HCl, $0{,}5H_2O$ | 266-269 | (-) |
| 50 | H | H | H | HCl | 270-271 | (+) |
| 51 | 4-OH | H | H | $H_2O$ | > 340 | (±) |
| 52 | 4-Phenyl | H | H | HCl | 240-242 | (±) |
| 53 | 2-Br | H | H | $1{,}5H_2O$ | 250-252 | (±) |
| 54 | 2-OH | H | H | $H_2O$ | 265-270 | (±) |

Die Herstellung einiger der erfindungsgemäß verwendbaren Verbindungen, sowie die Herstellung der notwendigen Ausgangsstoffe sind in der deutschen Offenlegungsschrift 36 12 337 auf die an dieser Stelle Bezug genommen wird, ausführlich beschrieben. Ein Verfahren zur Herstellung der Verbindungen ist dadurch gekennzeichnet, daß man eine Verbindung der Formel II

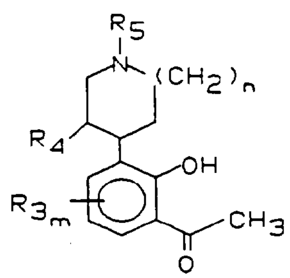

II

worin $R_4$ Hydroxy oder Acetoxy und $R_3$, $R_5$, n und m die genannten Bedeutungen haben, z.B. mit einem Alkalimetall und dem Alkylester einer Säure der Formel $R_1$-COOAlkyl, wobei $R_1$ die zur Formel I genannten Bedeutungen hat, umsetzt zu einem Diketon der Formel III

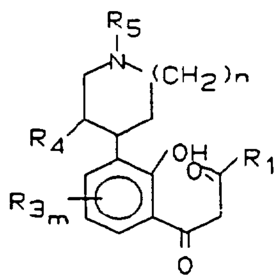

III

und die erhaltene Verbindung durch Umsetzen mit einer Mineralsäure zyklisiert zu einer Verbindung der Formel I

Die Bedingungen für die einzelnen Reaktionsschritte sind die gleichen wie in der deutschen Offenlegungsschrift 36 12 337 beschrieben. Eine besonders bevorzugte Methode zur Herstellung erfindungsgemäßer Verbindungen ist die Umsetzung einer Verbindung der Formel IV

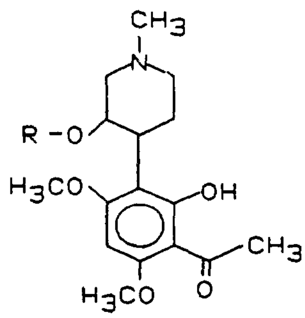

IV

in der R gleich H ist,
mit einer Verbindung der Formel

$$R_1\text{-COO X}$$

in der X= Wasserstoff oder Halogen, vorzugsweise Wasserstoff oder Chlor ist. Diese Veresterung findet unter allgemein bekannten Bedingungen statt, wie z.B. im Organikum, VEB, Deutscher Verlag der Wissenschaften, 15. Auflage, Berlin 1977, Kapitel D7. beschrieben. Die so gebildeten Ester werden mit Basen, z.B. Natriumhydrid, vorzugsweise in aprotischen Lösungsmitteln, z.B. Tetrahydrofuran, Dioxan oder N,N-Dimethylformamid, in einer Inertatmosphäre behandelt, wobei Diketone entstehen, die üblicherweise nicht isoliert werden. Die beschriebene Umsetzung wird in den Beispielen näher erläutert.

Die erfindungsgemäßen Verbindungen besitzen pharmakologische Eigenschaften, insbesondere inhibieren sie Onkogen-kodierte Kinasen, wie Tyrosinkinase, Serin/Threoninkinase, und Wachstumsfaktorrezeptortyrosinkinase und es kann deshalb erwartet werden, daß sie Wachstum und Ausbreitung von Tumoren inhibieren und in der Tumortherapie angewendet werden können.

Die erfindungsgemäße Verwendung der 4-H-1-Benzopyran-4-on Derivate erfolgt in an sich bekannter, dem Fachmann geläufiger Weise. Als Arzneimittel wird eine Wirksame Menge des genannten Wirkstoffs entweder per se oder vorzugsweise in Kombination mit geeigneten pharmazeutischen Hilfsstoffen in Form von Tabletten, Dragees, Kapseln, Suppositorien, Emulsionen, Suspensionen oder Lösungen eingesetzt, wobei der Wirkstoffgehalt bis etwa 95 %, vorzugsweise zwischen 10 und 75 % beträgt.

Welche Hilfsstoffe für die gewünschte Arzneimittelformulierung geeignet sind, ist dem Fachmann aufgrund seines Fachwissens geläufig. Neben Tablettenhilfsstoffen, Lösemitteln, Gelbildnern, Suppositoriengrundlagen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Der Wirkstoff kann oral, parenteral, intravenös oder rektal appliziert werden, wobei die intravenöse Applikation bevorzugt ist. Für eine orale Anwendungsform wird der genannte Wirkstoff gegebenenfalls mit weiteren aktiven Verbindungen mit den dafür geeigneten Zusatzstoffen, wie Trägerstoffen, Stabilisatoren oder inerten Verdünnungsmitteln, vermischt und durch die üblichen Methoden in geeignete Darreichungsformen gebracht, wie Tabletten, Dragees, Steckkapseln, sowie wäßrige, alkoholische oder ölige Suspensionen beziehungsweise Lösungen. Als inerte Träger können z.B. Gummi arabicum, Magnesia, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder Lösemittel kommen beispielsweise pflanzliche oder tierische Öle in Betracht, wie Sonnenblumenöl oder Lebertran.

Zur subkutanen oder intravenösen Applikation wird der Wirkstoff gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen zum Beispiel in Frage Wasser, physiologische Kochsalzlösung oder Alkohole zum Beispiel Ethanol, Propanol, Glycerin, daneben auch Zuckerlösungen wie Glucose- oder Mannitlösungen, oder auch eine Mischung aus den verschiedenen genannten Lösungsmitteln.

Die zu verabreichende Dosis an 4-H-1-Benzopyran-4-on Derivaten kann einen weiten Bereich überstreichen. Als täglich zu verabreichende Dosis ist eine solche zu wählen, die dem gewünschten Effekt angepaßt ist. Pro Patient werden vorzugsweise täglich etwa 20 bis 1000 mg, vorzugsweise intravenös verabreicht. Es können im Bedarfsfall auch darüber oder darunter liegende Tagesdosen verabreicht werden, wobei eine Höchstmenge von 2000 mg nur kurzzeitig überschritten werden sollte.

Die pharmakologischen Eigenschaften der genannten Verbindungen werden durch die folgenden pharmakologischen Tests belegt, welche mit erfindungsgemäßen Verbindungen und ihrer Salze ausgeführt wurden; die erhaltenen Ergebnisse sind in der Tabelle III angeführt.

**Prüfungsmethoden**

**Tyrosinkinase-Inhibitionstests**

Prüfungsverfahren:

Ausgangsmaterial für die Tyrosinkinase-Aktivität war die Ratten-Tumorzellinie RR 1022 (ATCC CCL47), die in RPMI 1640-Medium + 10 % FCS kultiviert wurde. Diese Zellinie ist mit RSV (Rous-Sarkom-Virus) transformiert und enthält das Onkogenprodukt $PP60^{v\text{-}src}$, welches Tyrosinkinase-Aktivität besitzt.

Die Zellen wurden bis fast zur Konfluenz kultiviert, mit PBS (Phosphate Buffered Saline) gewaschen, von der Kulturflasche abgeschabt und zweimal wiederholt gewaschen (0,85 % NaCl) und zentrifugiert (200 x g).

Schließlich wurden 100 µl Puffer (10 % Glyzerin, 25 mM Tris-HCl pH 7,4, 10 mM KCl, 1 mM EDTA, 1 % ®Triton X-100, Tensid der Fa. Rohm & Haas, Philadelphia, USA, 2 mM PMSF (Phenylmethyl sulfonyl fluorid), 100 Kallikrein inaktivierende Einheiten Aprotinin/ml, 2 mM Dithiotreitol) pro 1 x $10^6$ Zellen zugegeben, um die Zellen zu lysieren. Nach 5' bei 4°C wurde das Lysat 10' bei 10 000 x g zentrifugiert, und der Überstand wurde als Ausgangsmaterial für Tyrosinkinase-Aktivität verwendet.

Die Tyrosinkinase-Aktivität des Lysats wurde mit Poly (Glu, Tyr), 4:1, als Substrat gemessen. Der Inhibitor wurde mit Zellysat, Substrat (2 mg/ml) und $Mg^{2+}$ (10 mM) in 100 mM HEPES (N-2-Hydroxyethylpiperazin-N'-2-ethansulfonsäure), pH 7,2, vorinkubiert und die Reaktion durch Zugabe von $\gamma$-$^{32}P$ ATP (40 µM) begonnen. Nach 15' bei 30°C wurde das Substrat mit 10 % TCA (Trichloressigsäure) gefällt, auf eine Millititer Filtration Plate (Millipore Corporation, Mass., USA) filtriert, gewaschen und getrocknet. Der Einbau von $^{32}P$ wurde mittels eines Flüssigkeitsszintillationszählers ermittelt.

**Ergebnisse:**

Die Substanzen wurden bei einer Maximalkonzentration von 45 µg/ml geprüft und schrittweise 1:10 verdünnt. $IC_{50}$ bezeichnete die Konzentration, bei der 50 % der Ausgangsenzymaktivität gehemmt wurde (s. Tab. III).

**Test auf 3',5'-cAMP-abhängige Proteinkinasehemmung**

**Versuchsbeschreibung:**

Die katalytische Untereinheit der cAMP-abhängigen Proteinkinase (Sigma) wurde, wie von Sigma (Sigma Chemical Co., St. Louis, MO, USA) beschrieben, rekonstituiert. Die Enzymaktivität wurde mit Kemptid (Sigma) (Leu-Arg-Arg-Ala-Ser-Leu-Gly) als Substrat gemessen. Der Inhibitor wurde mit Enzym, Substrat (190 µM), $Mg^{2+}$ (5 mM), 0,25 mg/ml BSA und 3,75 mM Mercaptoäthanol in 50 mM MOPS (4-Morpholinpropansulfonsäure) pH 6,9, vorinkubiert. Die Reaktion wurde durch Zugabe von $\gamma$-$^{32}P$ ATP (40 µM) begonnen. Nach 15' bei 30°C wurde ein aliquoter Teil auf p81-Ionenaustauschpapier (2 x 2 cm; Whatman Paper Ltd. Großbritannien) gegeben, in 75 mM $H_3PO_4$ getaucht, gewaschen, getrocknet, und der Einbau von $^{32}P$ wurde mittels eines Flüssigkeitsszintillationszählers ermittelt.

**Ergebnisse:**

Ergebnisse sind als %-Hemmung der Ausgangsenzymaktivität bei einer Inhibitorkonzentration von 45 µg/ml ausgedrückt (s. Tab. III).

Tabelle III

| Formel Ic | | | | |
|---|---|---|---|---|
| worin $R_2 = R_8 = R_9 = H$ und $R_4 = OH$ | | | | |
| $R_1$ | X | Vorzeichen der optischen Drehung | $IC_{50}$ in µg/ml pp60 v-src | %-Hemmung c-AMP-abhängiger Proteinkinase bei 45 µg/ml Inhibitorkonzentration |
| 2-Bromphenyl | - | (±) | 22,3 | 63 |
| (2-Chlorphenyl)methyl | HCl | (±) | >45,0 | 69 |
| Phenyl | HCl | (-) | 5,7 | 46 |
| 2-Chlorphenyl | HCl | (+) | 9,2 | 27 |
| 2-Fluorphenyl | HCl | (±) | 28,0 | 0 |
| 4-Methylphenyl | HCl | (±) | 2,7 | 55 |
| 3-Pyridyl | HCl | (±) | 10,6 | 0 |
| 2-Pyridyl | HCl | (±) | 34,8 | 0 |
| Phenyl | HCl | (+) | 0,7 | 13 |
| 4-Pyridyl | HCl | (±) | 4,2 | 12 |
| n-Propyl | HCl | (±) | 45,0 | 0 |
| 2-Chlorphenyl | HCl | (±) | 3,3 | 29 |
| Äthyl | HCl | (±) | >45,0 | 0 |
| Phenyl | HCl | (±) | 1,9 | 48 |
| 4-Bromphenyl | HCl | (±) | 3,4 | 65 |
| n-Butyl | HCl | (±) | 0,0 | n.b. |
| 2-Pyridyl | HCl | (-) | 7,8 | 6 |
| 2-Naphthyl | HCl | (±) | 3,4 | 0 |
| 4-Fluorphenyl | HCl | (±) | 2,7 | 25 |
| 4-Chlorphenyl | HCl | (±) | 1,6 | 37 |
| n-Propyl | HCl | (-) | >45,0 | 5 |

Das folgende Beispiel erläutert die Erfindung.

SRC Tumor Test:

Einzelzellsuspensionen (Zellkultur A 549 oder vereinzelte humane Tumor Xenografts LXF 529) in Gewebekleber ((R)Beriplast, Behringwerke AG) wurden im Verhältnis 1:2 mit RPMI 1640 Medium verdünnt und mit 15 % FCS ergänzt zu einer Zellkonzentration von $10^7$ Zellen/50 µl. Diese Suspension wurde schnell in 50 µl Glaskapillaren gesaugt (Durchmesser 1,5 mm), deren innere Wände mit einer Thrombin/$CaCl_2$-Lösung angefeuchtet waren (500 Einheiten Thrombin in 1 ml 40 mmol $CaCl_2$).

Nach Verfestigung der Zell-Fibrin-Mischung (ungefähr 5 Min. bei Raumtemperatur) wurde mittels Luftdruck das Fibrinnetzwerk aus der Glaskapillare in eine Petri-Schale befördert.

Das Fibrinnetzwerk wurde in 2 mm große Teile zerschnitten (≈ $5.10^5$ Zellen pro Stück) und die Teile wurden bis zur Implantation in RPMI 1640 Medium, ergänzt mit 15 % FCS, aufbewahrt.

Ein einzelnes Fibrinstück wurde unter die Nierenkapsel ei-ner nackten Maus implantiert und anschließend wurden zwei zueinander senkrechte Durchmesser des implantierten Stückes mittels eines Mikroskops mit Okular-Mikrometer gemessen (Tag 0). Die Tumorgröße wurde gemessen nach der Formel

$$V = a \times b$$

V = Tumorgröße
a = größter Durchmesser
b = Durchmesser senkrecht zu a

Die Testsubstanz wurde i.v. oder p.o. täglich am Tag 2 - 15 in der maximal verträglichen Dosis (MVD) und einer Dosis, die 2/3 der MVD gemäß den Vorversuchen betrug, verabreicht. Es wurden 5 Mäuse/Gruppe herangezogen.

Am 21. Tag nach der Implantation wurden die Tiere geopfert, die Niere wurde herauspräpariert und die Tumorgröße wurde erneut gemessen. Die Wirksamkeit der Testsubstanz wurde anhand der Tumorwachstumshemmung bestimmt.

Die relative Tumorgröße wurde mittels folgender Formel bestimmt

$$V_R = \frac{V_t}{V_o}$$

$V_t$ = Tumorgröße am Ende des Experiments (Tag 21)
$V_o$ = Tumor-/Fibringröße am Tag der Implantation

Anschließend wurde die mittlere relative Tumorgröße der behandelten Gruppe ($V_T$) zu der entsprechenden mittleren Tumorgröße der Kontrollgruppe ($V_C$) gemäß folgender Formel in Beziehung gesetzt:

$$T/C\ \% = \frac{V_T}{V_C} \times 100$$

Die statistische Signifikanz ( p 0,05) der antitumoralen Wirksamkeit wurde mittels des Wilcoxon U Tests bestimmt. Tabelle IV zeigt die Testergebnisse.

**Tabelle IV**

| Testsubstanz | Tumor | Dosis (mg/kg/Tag) | Behandlungsdauer (Tage) | T/C (%) |
|---|---|---|---|---|
| | broncho-genes Karzinom | | | |
| Bsp. 17 | LXF 529 | 25 i.v. | 2-15 | 81 n.s. |
| | | 35 i.v. | 2-15 | 67 |
| | A549 | 35 i.v. | 2-15 | 70 |
| | | 100 p.o. | 2-15 | 73 |
| Bsp. 9 | LXF 529 | 2 i.v. | 2-15 | 59 |
| | | 4 i.v. | 2-15 | 51 |
| Bsp. 19 | LXF 529 | 100 i.v. | 2-15 | 98 n.s. |
| | | 150 i.v. | 2-15 | 80 |

n.s. = nicht signifikant ($p > 0.05$)

**Beispiel 1**

**(+/-)-cis-5,7-Dimethoxy-2-(2-thienyl)-8-[4-(3-acetoxy-1-methyl)piperidinyl]-4H-1-benzopyran-4-on**

Zu der Lösung von (±)-cis-3-Acetoxy-1-methyl-4-(3-acetyl-4,6-dimethoxy-2-hydroxy)phenylpiperidin der Formel IV, R = $COCH_3$, (3,0 g) in trockenem Pyridin (30 ml) wurde 2-Thiophencarbonsäure (2,73 g) bei 0°C gegeben, anschießend $POCl_3$ (2,2 ml). Die Reaktionsmischung wurde bei Zimmertemperatur zwei Stunden lang gerührt. Wasser (50 ml) wurde langsam zu der Reaktionsmischung dazugegeben, und später wurde die Reaktionslösung durch Zugabe von Natriumcarbonat alkalisch gemacht (pH 8). Die Reaktionsmischung wurde mit Essigester extrahiert (40 ml x 3). Die vereinigten organischen Extrakte wurde mit Wasser gewaschen, über wasserfreiem Natriumsulfat getrocknet, und das Lösungsmittel unter Vakuum entfernt. Der Rückstand wurde auf Silicagel chromatographiert (4 % MeOH in $CHCl_3$), Ausbeute

2,7 g (±)-cis-3-Acetoxy-1-methyl-4-(3-acetyl-4,6-dimethoxy-2-(2-thienyloxy)phenyl)piperidin, Schmelzpunkt 153-154°C.

Diese Verbindung wurde in trockenem Dioxan (50 ml) aufgenommen, Natriumhydrid (5 Äquivalente) wurde zugegeben, und die Mischung wurde vier Stunden lang bei 40°C gerührt. MeOH (10 ml) wurde zugegeben, um überschüssiges Natriumhydrid zu zerstören, und trockenes HCl-Gas wurde eingeleitet, bis der pH der Lösung deutlich sauer war. Die Reaktionsmischung wurde durch Zugabe von eiskalter Natriumcarbonat-Lösung aufgearbeitet, und das erhaltene Festprodukt wurde durch Filtration abgetrennt. Die weitere Reinigung wurde mittels Säulenchromatographie durchgeführt (Silicagel, 2 % MeOH, 1 % $NH_4OH$ in $CHCl_3$), und (±)-cis-5,7-Dimethoxy-2-(2-thienyl)-8-[4-(3-acetoxy-1-methyl)-piperidinyl]-4H-1-benzopyran-4-on wurde erhalten. Ausbeute 2,50 g, S.P. 207-208°C.

Analog Beispiel 1 wurden die folgenden Verbindungen hergestellt.

**Beispiel 2**

(±)-cis-5,7-Dihydroxy-2-(phenyl)-8-[4-(3-hydroxy-1-methyl)piperidinyl]-4H-1-benzopyran-4-on-hydrochlorid.

**Beispiel 3**

(-)-cis-5,7-Dihydroxy-2-phenyl-8-[4-(3-hydroxy-1-methyl)-piperidinyl]-4H-1-benzopyran-4-on-hydrochlorid.

**Beispiel 4**

(+)-cis-5,7-Dihydroxy-2-phenyl-8-[4-(3-hydroxy-1-methyl)-piperidinyl]-4H-1-benzopyran-4-on-hydrochlorid.

**Beispiel 5**

(-)-cis-5,7-Dihydroxy-2-(2-pyridyl)-8-[4-(3-hydroxy-1-methyl)piperidinyl]-4H-1-benzopyran-4-on-hydrochlorid.

**Beispiel 6**

(±)-cis-5,7-Dihydroxy-2-(2-pyridyl)-8-[4-(3-hydroxy-1-methyl)piperidinyl]-4H-1-benzopyran-4-on-hydrochlorid.

**Beispiel 7**

(±)-cis-5,7-Dihydroxy-2-(3-pyridyl)-8-[4-(3-hydroxy-1-methyl)piperidinyl]-4H-1-benzopyran-4-on-hydrochlorid.

**Beispiel 8**

(±)-cis-5,7-Dihydroxy-2-(4-pyridyl)-8-[4-(3-hydroxy-1-methyl)piperidinyl]-4H-1-benzopyran-4-on-hydrochlorid.

**Beispiel 9**

(-)-cis-5,7-Dihydroxy-2-(2-chlorphenyl)-8-[4-(3-hydroxy-1-methyl)piperidinyl]-4H-1-benzopyran-4-on-hydrochlorid.

**Beispiel 10**

(+)-cis-5,7-Dihydroxy-2-(2-chlorphenyl)-8-[4-(3-hydroxy-1-methyl)piperidinyl]-4H-1-benzopyran-4-on-hydrochlorid.

**Beispiel 11**

(±)-cis-5,7-Dihydroxy-2-(2-chlorphenyl)-8-[4-(3-hydroxy-1-methyl)piperidinyl]-4H-1-benzopyran-4-on-hydrochlorid.

**Beispiel 12**

(±)-cis-5,7-Dihydroxy-2-(2,5-dichlorphenyl)-8-[4-(3-hydroxy-1-methyl)piperidinyl]-4H-1-benzopyran-4-on-hydrochlorid.

**Beispiel 13**

(±)-cis-5,7-Dihydroxy-2-(2,4-dichlorphenyl)-8-[4-(3-hydroxy-1-methyl)piperidinyl]-4H-1-benzopyran-4-on-hydrochlorid.

**Beispiel 14**

(±)-cis-5,7-Dihydroxy-2-(4-methylphenyl)-8-[4-(3-hydroxy-1-methyl)piperidinyl]-4H-1-benzopyran-4-on-hydrochlorid.

**Beispiel 15**

(±)-cis-5,7-Dihydroxy-2-(3-methylphenyl)-8-[4-(3-hydroxy-1-methyl)piperidinyl]-4H-1-benzopyran-4-on-hydrochlorid.

**Beispiel 16**

(±)-cis-5,7-Dihydroxy-2-(4-aminophenyl)-8-[4-(3-hydroxy-1-methyl)piperidinyl]-4H-1-benzopyran-4-on-hydrochlorid.

**Beispiel 17**

(±)-cis-5,7-Dihydroxy-2-(3-bromphenyl)-8-[4-(3-hydroxy-1-methyl)piperidinyl]-4H-1-benzopyran-4-on-hydrochlorid.

**Beispiel 18**

(±)-cis-5,7-Dihydroxy-2-(2-naphthyl)-8-[4-(3-hydroxy-1-methyl)piperidinyl]-4H-1-benzopyran-4-on-hydrochlorid.

**Beispiel 19**

(±)-cis-5,7-Dihydroxy-2-(4-hydroxyphenyl)-8-[4-(3-hydroxy-1-methyl)piperidinyl]-4H-1-benzopyran-4-on.

**Beispiel 20**

(±)-cis-5,7-Dihydroxy-2-(2-hydroxyphenyl)-8-[4-(3-hydroxy-1-methyl)piperidinyl]-4H-1-benzopyran-4-on.

**Beispiel 21**

Wirkstofflösungen, die zur Injektion geeignet sind, enthalten die nachfolgend genannten Bestandteile und können auf an sich bekannte Weise hergestellt werden, indem die Stoffe miteinander vermischt und in sterile Ampullen abgefüllt werden. Die Injektionslösungen dienen zur Antitumor-Behandlung in einer Dosis von 1-2 Injektionseinheiten (1 Injektionseinheit = 1 Ampulle) pro Tag.

| Bestandteile (pro Ampulle) | Gewicht |
|---|---|
| (±)-(cis-5,7-Dimethoxy-2-(2-thienyl)-8-[4-(3-acetoxy-1-methyl)piperidinyl] -4H-1-benzopyran-4-on | 200 mg |
| Natriumchlorid | 50 mg |
| Methylparaben | 5 mg |
| steriles Wasser | 5 mg |

**Patentansprüche**

1. Verwendung von Verbindungen der Formel I

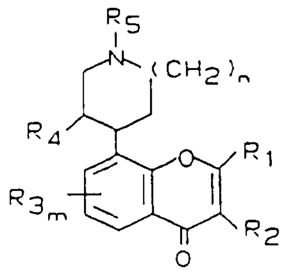

I

in der

$R_1$ Wasserstoff, Alkyl mit 1 bis 6 Kohlenstoffatomen, Aryl-$C_1$-$C_4$-alkyl, durch Halogen, Trifluormethyl, Hydroxy oder Carboxy substituiertes $C_1$-$C_6$-Alkyl, $C_3$-$C_6$-Cycloalkyl, einen $C_3$-$C_9$-Heterocyclus mit 1, 2 oder 3 Heteroatomen wie N, S, O oder deren beliebige Kombinationen, $C_3$-$C_6$-Cycloalkyl-$C_1$-$C_4$-alkyl, $C_2$-$C_6$-Alkenyl, $C_2$-$C_6$-Alkinyl, polycyclische Ringe einbegriffen, aromatische heterocyclische Reste, ggb. durch Halogen, $C_1$-$C_4$-Alkyl, $C_1$-$C_4$ Alkoxy, Hydroxy, Carboxy, COO-Alkyl, $CONH_2$, CONH-Alkyl, $CON(Alkyl)_2$, Nitro, Trifluoromethyl, Amino, $C_1$-$C_4$ Alkylamino, Di-$C_1$-$C_4$ alkylamino, aromatische Heterocyclen wie Pyridylgruppen und polycyclische aromatische Reste wie Naphthylgruppen. substituiertes Aryl, Carboxyl oder eine Aldehyd- oder COO-$C_1$-$C_4$-Alkylgruppe, eine primäre Amino-, Alkylamino-, Aralkylamino-, Dialkylamino-, Amido-, Arylamino-, Diarylaminogruppe oder -$CH_2O$-$C_1$-$C_4$-Alkyl bedeutet;
$R_2$ Wasserstoff oder $C_1$-$C_3$-Alkyl bedeutet;
$R_3$ gleich ist und OH oder $OCH_3$ bedeutet;
$R_4$ Hydroxy bedeutet;
$R_5$ $CH_3$ bedeutet;
m gleich der Zahl 2 ist und
n gleich der Zahl 1 ist,

oder deren pharmakologisch unbedenklichen Säureadditionssalzen zur Herstellung eines Arzneimittels mit einer Wirkung gegen Tumoren.

**2.** Verwendung von Verbindungen gemäß Anspruch 1 mit der Formel Ia

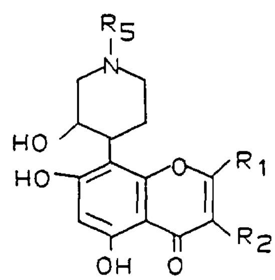

Ia

in der $R_1$ Wasserstoff oder $C_1$-$C_3$-Alkyl, Naphthyl, ggb. durch Halogen, $C_1$-$C_4$-Alkyl, $C_1$-$C_4$ Alkoxy, Hydroxy, Carboxy, COO-Alkyl, $CONH_2$, CONH-Alkyl, $CON(Alkyl)_2$, Nitro, Trifluoromethyl, Amino, $C_1$-$C_4$ Alkylamino, Di-$C_1$-$C_4$ alkylamino, aromatische Heterocyclen wie Pyridylgruppen und polycyclische aromatische Reste wie Naphthylgruppen. substituiertes Aryl oder einen $C_3$-$C_9$-Heterocyclus bedeutet;

$R_2$ Wasserstoff oder $C_1$-$C_3$-Alkyl bedeutet und

$R_5$ $CH_3$ bedeutet,
oder deren pharmakologisch unbedenklichen Säureadditionssalzen.

**3.** Verwendung von Verbindungen der Formel Ia gemäß Anspruch 2, dadurch gekennzeichnet, daß $R^1$ Phenyl, Thienyl, Pyridyl, Chlorphenyl, Dichlorphenyl, Methylphenyl, Aminophenyl, Bromphenyl, Hydroxyphenyl oder Napthyl bedeutet,

$R^2$ Wasserstoff bedeutet, und

$R^5$ Methyl bedeutet oder deren pharmakologisch unbedenklichen Säureadditionssalzen.

## Claims

1. The use of a compound of the formula I

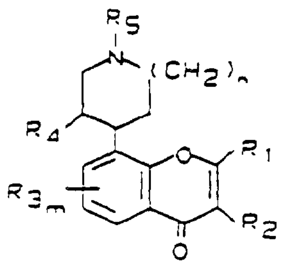

I

in which

$R_1$ is hydrogen, alkyl having 1 to 6 carbon atoms, aryl-$C_1$-$C_4$-alkyl, $C_1$-$C_6$-alkyl which is substituted by halogen, trifluoromethyl, hydroxyl or carboxyl, $C_3$-$C_6$-cycloalkyl, a $C_3$-$C_9$-heterocyclic ring having 1, 2 or 3 hetero atoms, such as N, S, O or any combinations thereof, or else $C_3$-$C_6$-cycloalkyl-$C_1$-$C_4$-alkyl, $C_2$-$C_6$-alkenyl, $C_2$-$C_6$-alkynyl, polycyclic rings included, or aromatic heterocyclic radicals, aryl which is optionally substituted by halogen, $C_1$-$C_4$-alkyl, $C_1$-$C_4$-alkoxy, hydroxyl, carboxyl, COO-alkyl, $CONH_2$, CONH-alkyl, $CON(alkyl)_2$, nitro, trifluoromethyl, amino $C_1$-$C_4$-alkylamino, di-$C_1$-$C_4$-alkylamino, aromatic heterocyclic rings, such as pyridyl groups, and polycyclic aromatic radicals, such as naphthyl groups, or is carboxyl or an aldehyde or COO-$C_1$-$C_4$-alkyl group, a primary amino, alkylamino, aralkylamino, dialkylamino, amido, arylamino or diarylamino group, or $-CH_2O$-$C_1$-$C_4$-alkyl;
$R_2$ is hydrogen or $C_1$-$C_3$-alkyl;
$R_3$ radicals are identical and are OH or $OCH_3$;
$R_4$ is hydroxyl;
$R_5$ is $CH_3$;
m is the number 2 and
n is the number 1,
or of a pharmacologically acceptable acid addition salt thereof, for the preparation of a pharmaceutical having an antitumor action.

2. The use of a compound as claimed in claim 1, of the formula Ia

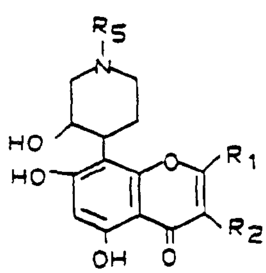

Ia

in which $R_1$ is hydrogen or $C_1$-$C_3$-alkyl, naphthyl, aryl which is optionally substituted by halogen, $C_1$-$C_4$-alkyl, $C_1$-$C_4$-alkoxy, hydroxyl, carboxyl, COO-alkyl, $CONH_2$, CONH-alkyl, $CON(alkyl)_2$, nitro, trifluoromethyl, amino $C_1$-$C_4$-alkylamino, di-$C_1$-$C_4$-alkylamino, aromatic heterocyclic rings, such as pyridyl groups, and polycyclic aromatic radicals, such as naphthyl groups or a $C_3$-$C_9$-heterocyclic ring;
$R_2$ is hydrogen or $C_1$-$C_3$-alkyl and
$R_5$ is $CH_3$,
or of a pharmacologically acceptable acid addition salt thereof.

3. The use of a compound of the formula Ia as claimed in claim 2, in which $R_1$ is phenyl, thienyl, pyridyl, chlorophenyl, dichlorophenyl, methylphenyl, aminophenyl, bromophenyl, hydroxyphenyl or naphthyl,

$R_2$ is hydrogen and
$R_5$ is methyl,
or a pharmacologically acceptable acid addition salt thereof.

## Revendications

**1.** Utilisation de composés de formule I

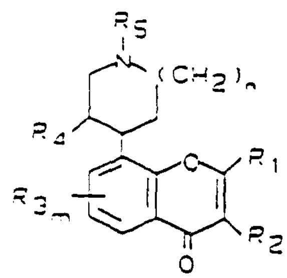

I

dans laquelle

$R_1$ représente hydrogène, alkyle en $C_1$-$C_6$, aryl-$C_1$-$C_4$-alkyle, $C_1$-$C_6$-alkyle substitué par halogène, trifluorométhyle, hydroxyle ou carboxy, $C_3$-$C_6$-cycloalkyle, un hétérocycle en $C_3$-$C_9$ avec 1, 2 ou 3 hétéroatomes tels que N, S, O ou une combinaison quelconque de ceux-ci, $C_3$-$C_6$-cycloalkyl-$C_1$-$C_4$-alkyle, $C_2$-$C_6$-alcényle, $C_2$-$C_6$-alcynyle, y compris des noyaux polycycliques, des résidus hétérocycliques aromatiques, aryle éventuellement substitué par halogène, $C_1$-$C_4$-alkyle, $C_1$-$C_4$-alcoxy, hydroxy, carboxy, COO-alkyle, $CONH_2$, CONH-alkyle, $CON(alkyl)_2$, nitro, trifluorométhyle, amino, $C_1$-$C_4$-alkylamino, di-$C_1$-$C_4$-alkylamino, hétérocycles aromatiques tels que des groupes pyridyle ou des résidus polycycliques aromatiques tels que des groupes naphtyle, carboxyle ou un groupe aldéhyde ou COO-$C_1$-$C_4$-alkyle, un groupe amino primaire, alkylamino, aralkylamino, dialkylamino, amido, arylamino, diarylamino ou -$CH_2$-O-$C_1$-$C_4$-alkyle;
$R_2$ représente l'hydrogène ou $C_1$-$C_3$-alkyle; tous les groupes $R_3$ sont indentiques et représentent OH ou $OCH_3$;
$R_4$ représente hydroxy;
$R_5$ représente $CH_3$;
m vaut le nombre 2 et
n vaut le nombre 1,
ou leurs sels d'addition pharmaceutiquement acceptables avec un acide pour la préparation d'un médicament présentant une activité anti-tumorale.

**2.** Utilisation de composés selon la revendication 1 de formule Ia

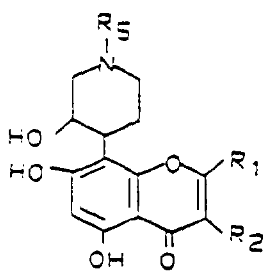

Ia

dans laquelle $R_1$ représente hydrogène ou $C_1$-$C_3$-alkyle, naphtyle, aryle éventuellement substitué par halogène, $C_1$-$C_4$-alkyle, $C_1$-$C_4$-alcoxy, hydroxy, carboxy, COO-alkyle, $CONH_2$, CONH-alkyle, $CON(alkyl)_2$, nitro, trifluorométhyle, amino, $C_1$-$C_4$-alkylamino, di-$C_1$-$C_4$-alkylamino, hétérocycles aromatiques tels que des groupes pyridyle ou des résidus polycyliques aromatiques tels que des groupes naphtyle, ou un hétérocycle en $C_3$-$C_9$;

$R_2$ représente hydrogène ou $C_1$-$C_3$-alkyle et
$R_5$ représente $CH_3$
ou leurs sels d'addition pharmaceutiquement acceptables avec un acide.

**3.** Utilisation de composés de formule Ia selon la revendication 2, caractérisée en ce que $R_1$ représente phényle,

thiényle, pyridyle, chlorophényle, dichlorophényle, méthylphényle, aminophényle, bromophé-nyle, hydroxyphényle ou naphtyle;

$R_2$ représente hydrogène et
$R_5$ représente méthyle, ou leurs sels d'addition pharmaceutiquement acceptables avec un acide.